# EUROPÄISCHE PATENTSCHRIFT

(11) **EP 0 682 012 B1**
(45) Veröffentlichungstag und Bekanntmachung des Hinweises auf die Patenterteilung: **28.10.1998**
(21) Anmeldenummer: 95106226.4
(22) Anmeldetag: 26.04.1995
(51) Int. Cl.: C07C 273/18, C07C 275/60, C08G 18/78

(54) **Verfahren zur Herstellung von Allophanatgruppen aufweisenden lichtechten Polyisocyanaten**
Process for the preparation of light stable polyisocyanates containing allophanate groups
Procédé pour la préparation des polyisocyanates solides à la lumière qui contiennent les groupes allophanates

(30) Priorität: 09.05.1994 DE 4416321
(43) Veröffentlichungstag der Anmeldung: 15.11.1995
(73) Patentinhaber: BAYER AG, 51368 Leverkusen (DE)
(72) Erfinder: Mertes, Harald, Dr., D-50670 Köln (DE); Pedain, Josef, Dr., D-51061 Köln (DE); Halpaap, Reinhard, Dr., D-51519 Odenthal (DE)

(56) Entgegenhaltungen:
- EP-A- 0 000 016
- EP-A- 0 000 194
- EP-A- 0 016 355
- EP-A- 0 031 650
- EP-A- 0 303 150
- EP-A- 0 393 903
- GB-A- 994 890
- US-A- 4 738 991
- CHEMICAL ABSTRACTS, vol. 100, no. 12, 19. März 1984, Columbus, Ohio, US; abstract no. 86239c, Z. WIRPSZA ET. AL. 'Urea amidation of compounds having active hydrogen atoms. Urea amidation of polyethylene glycols.' Seite 7 ;Spalte 2 ; & PRZEM. CHEM., Bd.62, Nr.12, 1983 Seiten 679 - 81

## Beschreibung

Die vorliegende Erfindung betrifft ein neues Verfahren zur Herstellung von hellfarbigen lichtechten Allophanatgruppen aufweisenden (cyclo)aliphatischen Polyisocyanaten, sowie die Verwendung der nach diesem Verfahren erhältlichen Polyisocyanate als Aufbaukomponente bei der Herstellung von Polyurethankunststoffen.

Verfahren zur Herstellung von Allophanatgruppen aufweisenden Polyisocyanaten sind bereits beschrieben in der GB-PS 994 890, US-PS 3 769 318, EP-B 0 000 016, EP-B 0 000 194 und EP-A-0 303 150.

Gemäß GB-PS 994 890 werden Urethangruppen aufweisende Polyisocyanate aus einfachen ein- oder mehrwertigen Alkoholen und organischen Polyisocyanaten insbesondere Diisocyanaten durch mehrstündiges Erhitzen auf erhöhte Temperaturen bzw. in Gegenwart von Katalysatoren wie Metallcarboxylaten, Metallchelaten oder tertiären Aminen mit weiteren Mengen an organischen Polyisocyanaten vorzugsweise Diisocyanaten umgesetzt, bis der für vollständige Umsetzung der Urethangruppen berechnete Isocyanat-Gehalt erreicht ist. Die exakte Konstitution der Reaktionsprodukte kann gemäß der Lehre der britischen Patentschrift nicht mit Sicherheit angegeben werden. Aus den gemessenen NCO-Werten der Reaktionsgemische bzw. der daraus isolierten Endprodukte wird geschlossen, daß diese im wesentlichen Allophanatpolyisocyanate darstellen.

Wie in einem Vergleichsbeispiel gezeigt wurde, werden bei rein thermischer Reaktionsführung gemäß GB-PS 994 890 stark gefärbte Produkte erhalten, die nach ¹³C-NMR-Spektroskopie nicht nur Allophanatpolyisocyanate, sondern auch beträchtliche Mengen an Uretdion-, Harnstoff- und Biuretpolyisocyanaten, die durch Nebenreaktionen wie beispielsweise Dimerisierung und Biuretisierung entstehen, sowie nicht umgesetzte Urethane enthalten. Dies ist dadurch zu erklären, daß beim Abstoppen der Reaktion bei dem für eine vollständige All.ophanatisierung berechneten NCO-Gehalt für jede durch Nebenreaktion abreagierte NCO-Gruppe eine Urethangruppe unu mgesetzt im Reaktionsgemisch zurückbleibt.

Die in der GB-PS 994 890 beschriebenen Katalysatoren wie Metallcarboxylate, Metallchelate und tertiäre Amine sind seit langem als Dimerisierungs- und/oder Trimerisierungskatalysatoren für Isocyanate bekannt, so daß das Auftreten solcher Nebenreaktionen in beträchtlichem Ausmaß bei der Umsetzung von Urethangruppen mit Isocyanaten zu Allophanaten, wie in den Vergleichsbeispielen 3 und 4 gezeigt, durchaus verständlich wird. Es ist der Patentschrift nicht zu entnehmen, unter welchen Bedingungen und/oder mit Hilfe welcher Katalysatoren reine Allophanatpolyisocyanate hergestellt werden können. Ebensowenig kann abgeleitet werden, unter welchen Bedingungen farbhelle lichtechte Produkte erhalten werden können.

Das Problem, Allophanatpolyisocyanate herzustellen, die nicht von dimeren und trimeren Polyisocyanaten begleitet sind, wird in der US-PS 3 769 318 behandelt. Gemäß dieser Patentschrift werden Allophanatgruppen aufweisende Polyisocyanate, die mindestens eine aromatisch gebundene Isocyanatgruppe aufweisen, durch Umsetzung N-substituierter Carbamidsäureester mit Isocyanaten in Gegenwart von alkylierend wirkenden Schwefelsäure- oder Sulfonsäureestern hergestellt. Gemäß einer besonderen Variante erfolgt die Durchführung dieses Verfahrens in Gegenwart von bestimmten Metallverbindungen. In der langen diesbezüglichen Liste erwähnt die Beschreibung auch Zinn(II)-octoat, jedoch wird dieses Salz in keinem konkreten Ausführungsbeispiel eingesetzt. Die Vorveröffentlichung vermittelt somit keinerlei Hinweis, daß zur Herstellung von rein (cyclo)aliphatischen, Allophanatgruppen aufweisenden Polyisocyanaten Zinnverbindungen der nachstehend beispielhaft genannten Art und nur diese aus der langen Liste geeigneter Metallverbindungen auszuwählen sind.

In der EP-B 0 000 016 und EP-A 0 303 150 wird rein thermische Umsetzung von Urethanen mit organischen Polyisocyanaten zu Allophanatpolyisocyanaten beschrieben. Wie aus Vergleichsbeispiel 1 ersichtlich, führen rein thermische Umsetzungen von Urethanen und Isocyanaten jedoch zu Nebenreaktionen in beträchtlichem Ausmaß sowie zur Verfärbung der Produkte.

In der EP-B 0 000 194 wird ein Verfahren zur Herstellung von Allophanatpolyisocyanaten mit aliphatisch und/oder cycloaliphatisch gebundenen Isocyanatgruppen beschrieben, bei dem Urethangruppen enthaltende Verbindungen in Gegenwart starker Säuren mit Polyisocyanaten mit aliphatisch und/oder cycloaliphatisch gebundenen Isocyanatgruppen umgesetzt werden. Durch die Gegenwart starker Säuren wie beispielsweise Chlorwasserstoff werden Nebenreaktionen wie Trimerisierung und Biuretisierung weitgehend unterdrückt, die erhaltenen Produkte sind jedoch verfärbt und neigen beim längeren Stehen zum Nachdunkeln (Vergleichsbeispiel 2).

Als technologischer Hintergrund sind weiterhin in diesem Zusammenhang zu nennen die US-A 4738991, die EP-A 303150, 393903, 016355 und 000016. Bei diesen Literaturstellen werden ausgewählte Zinnsalze teils zur Urethanisierungsreaktionen oder Vernetzungsreaktionen eingesetzt. Zur Allophanatisierung alleine werden sie jedoch nicht eingesetzt.

Dem zitierten Stand der Technik sind somit keinerlei Anregungen zu entnehmen, wie hochwertige, insbesondere lichtechte, d.h. (cyclo)aliphatische Allophanatpolyisocyanate zugänglich sein könnten. Da jedoch davon ausgegangen werden mußte, daß derartige Polyisocyanate wertvolle Ausgangsmaterialien zur Herstellung von lichtechten Polyurethanlacken darstellen, war es die der Erfindung zugrunde liegende Aufgabe, ein Verfahren zur Herstellung derartiger Allophanatpolyisocyanate zur Verfügung zu stellen.

Überraschenderweise konnte diese Aufgabe dadurch gelöst werden, daß man die Umsetzung zwischen aliphatischen bzw. cycloaliphatischen Polyisocyanaten und Urethangruppen aufweisenden Verbindungen in Gegenwart von bestimmten, nachstehend näher beschriebenen Zinnverbindungen durchführt. Die erfindungsgemäßen Verfahrensprodukte zeichnen sich durch eine niedrige Farbzahl hohe Lichtechtheit und Farbstabilität und darüber hinaus durch eine vergleichsweise niedrige Viskosität aus und stellen wertvolle Ausgangsmaterialien zur Herstellung von Polyurethankunststoffen insbesondere Polyurethanlacken dar.

Gegenstand der vorliegenden Erfindung ist ein Verfahren zur Herstellung von aliphatisch und/oder cycloaliphatisch gebundene Isocyanatgruppen aufweisenden

Allophanaten durch Umsetzung von Urethangruppen aufweisenden organischen Verbindungen mit organischen Polyisocyanaten mit aliphatisch und/oder cycloaliphatisch gebundenen Isocyanatgruppen, bei einer Temperatur von 80 - 140°C, wobei das Allophanatgruppen enthaltende hergestellte Polyisocyanat durch Dünnschichtdestillation oder fraktionierte Extraktion von überschüssigem Diisocyanat befreit wird, dadurch gekennzeichnet, daß diese in Gegenwart von Zinn(II)-salzen, Zinn(II)-halogeniden und Zinn(II)-salzen organischer Säuren hergestellt wurden.

Gegenstand der vorliegenden Erfindung ist auch die Verwendung der nach diesem Verfahren erhältlichen Polyisocyanate als Aufbaukomponente bei der Herstellung von Polyurethankunststoffen, insbesondere als, gegebenenfalls in blockierter Form einzusetzendes, Vernetzerharz für Zweikomponenten-Polyurethanlacke.

Ausgangsmaterialien für das erfindungsgemäße Verfahren sind (i) Urethangruppen aufweisende organische Verbindungen und (ii) organische Polyisocyanate mit aliphatisch und/oder cycloaliphatisch gebundenen Isocyanatgruppen.

Bei den beim erfindungsgemäßen Verfahren einzusetzenden, Urethangruppen aufweisenden Verbindungen handelt es sich um beliebige, gegebenenfalls Isocyanatgruppen aufweisende Verbindungen, die 1 bis 70, vorzugsweise 1 bis 40 Gew.-% an Urethangruppen (berechnet als CHNO₂, Molekulargewicht = 59) aufweisen, und die vorzugsweise außer den Urethangruppen keine weiteren H-aktiven, gegenüber Isocyanatgruppen reaktionsfähigen Gruppen aufweisen. Geeignet sind beispielsweise Urethangruppen aufweisende Verbindungen, die durch Umsetzung von primäre Aminogruppen aufweisenden Aminen mit Chlorameisensäureestern erhalten worden sind. Bevorzugt handelt es sich bei den Urethangruppen aufweisenden Verbindungen jedoch um Umsetzungsprodukte von Isocyanaten, insbesondere Polyisocyanaten mit organischen Hydroxylverbindungen, d.h. Alkoholen oder Phenolen, vorzugsweise Alkoholen.

Gemäß einer besonderen Ausführungsform des erfindungsgemäßen Verfahrens werden als Ausgangsmaterialien in situ aus Phenolen oder Alkoholen und überschüssigen Mengen an aliphatischen bzw. cycloaliphatischen Polyisocyanaten hergestellte Urethane eingesetzt. Das bei dieser Umsetzung erhaltene Reaktionsgemisch enthält dann bereits die zweite Hauptkomponente des erfindungsgemäßen Verfahrens, das aliphatische bzw. cycloaliphatische Polyisocyanat, welches bei der Urethanherstellung im Überschuß eingesetzt worden war. Zu den bevorzugten, beim erfindungsgemäßen

Verfahren als Ausgangsmaterialien einzusetzenden Urethangruppen aufweisenden Verbindungen gehören solche der allgemeinen Formel 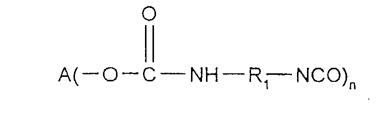 in welcher
- A: für einen Rest steht, wie er durch Entfernung der Hydroxylgruppen aus einer n-wertigen organischen, Hydroxylgruppen aufweisenden Verbindung erhalten wird, die außer den Hydroxylgruppen keine weiteren gegenüber Isocyanatgruppen reaktive Gruppen aufweist,
- R₁: für einen Rest steht, wie er durch Entfernung der Isocyanatgruppen aus einem Diisocyanat mit aliphatisch und/oder cycloaliphatisch gebundenen Isocyanatgruppen erhalten wird und
- n: für eine ganze Zahl von 1 bis 4 steht,
bzw. um Gemische von Urethangruppen aufweisenden Verbindungen der genannten Formel mit bis zu 50 Gew.-%, bezogen auf Gesamtgemisch, an höheren, durch Kettenverlängerungsreaktionen entstandenen Homologen dieser Verbindungen.

Diese besonders bevorzugt beim erfindungsgemäßen Vefahren einzusetzenden, Isocyanatgruppen aufweisenden Urethane enthalten im allgemeinen 1 bis 40 Gew.-% an Urethangruppen und 1 bis 30 Gew.-% an Isocyanatgruppen, wobei es sich bei den Resten A und R₁ um (cyclo)aliphatische Kohlenwasserstoffreste handelt.

Entsprechend diesen Ausführungen werden die bevorzugt beim erfindungsgemäßen Verfahren einzusetzenden, Isocyanatgruppen aufweisenden Urethane der genannten allgemeinen Formel vorzugsweise durch Umsetzung von Hydroxylgruppen aufweisenden Verbindungen der Formel

A(OH)ₙ

mit Diisocyanaten der Formel

R₁(NCO)₂

erhalten, wobei die Reaktionspartner in einem, einem NCO/OH-Äquivalentverhältnis von mindestens 1,1:1, vorzugsweise mindestens 1,8:1 und besonders bevorzugt 2:1 bis 24:1 entsprechenden Mengenverhältnissen zum Einsatz gelangen.

Ebenfalls möglich, jedoch weniger bevorzugt, wäre der Einsatz von Urethangruppen aufweisenden Verbindungen, die durch Umsetzung von Hydroxyl.gruppen aufweisenden Verbindungen A(OH)ₙ mit Monoisocyanaten und/oder höher als difunktionellen Polyisocyanaten gegebenenfalls im Gemisch mit Diisocyanaten erhalten worden sind, und welche gegebenenfalls keine freien Isocyanatgruppen aufweisen.

Die Herstellung der Urethangruppen aufweisenden Ausgangsmaterialien für das erfindungsgemäße Verfahren erfolgt nach den altbekannten Methoden der Polyurethanchemie, d.h. insbesondere durch einfaches Erhitzen der Ausgangsmaterialien auf 40 bis 150°C, vorzugsweise 50 bis 100°C.

Gegebenenfalls kann die Reaktion unter Katalyse mit den bekannten Urethanisierungskatalysatoren durchgeführt werden, bevorzugt erfolgt sie jedoch katalysatorfrei oder unter Verwendung solcher Katalysatoren, wie sie, wie nachfolgend beschrieben, für die Allophanatisierungsreaktion eingesetzt werden.

Als Polyhydroxylverbindungen A(OH)ₙ können sowohl Phenole wie z. B. Phenol, α-Naphthol, Kresol, Resorcin oder Trishydroxybenzole als auch alkoholische Hydroxylgruppen aufweisende organische Verbindungen eingesetzt werden. Derartige alkoholische Hydroxylgruppen aufweisende Verbindungen sind gegenüber den beispielhaft genannten Phenolen bevorzugt.

Zu diesen bevorzugten alkoholischen Hydroxylverbindungen A(OH)ₙ gehören
1. Niedermolekulare, gegebenenfalls Etherbrücken aufweisende 1-4-wertige aliphatische Alkohole des Molekulargewichtsbereichs 32 - 250 wie z. B. Methanol, Ethanol, Propanol, Isopropanol, Allylalkohol, isomere Butanole, Pentanole, Hexanole und Heptanole, 2-Ethylhexanol, Fettalkohole mit 10 - 20 Kohlenstoffatomen, Ethandiol, 1,2- und 1,3-Propandiol, 1,2-,1,3- und 1,4-Butandiol, 1,5-Pentandiol, Neopentylglykol, 1,6- und 2,5-Hexandiol, 3-Methyl-1,5-pentandiol, 2-Methyl-2-propyl-1,3-propandiol, 2,2-Diethyl-1,3-propandiol, 2-Ethyl-1,3-hexandiol, 2,2,4-Trimethyl-1,3-pentandiol, Trimethyl-1,6-hexandiol, 1,10-Decandiol, 1,12-Dodecandiol, 2-Methyl-1,4-butandiol, 2-Methyl-1,3-propandiol, Glycerin, Butantriol, 2-Hydroxymethyl-2-methyl-1,3-propandiol, 1,2,6-Hexantriol, Trimethylolethan, Trimethylolpropan, Pentaerythrit, Ethylenglykolmonoalkyl- oder arylether, Propylenglykolmonoalkylether, Diethylenglykol, Triethylenglykol, Tetraethylenglykol,
2. Cycloaliphatische 1-4-wertige Alkohole des Molekulargewichtsbereichs 86 - 250, wie z. B. Cyclopentanol, Cyclohexanol, Methylcyclohexanol, Trimethylcyclohexanol, 4-tert.-Butylcyclohexanol, Menthol, Borneol und Isoborneol, 2-Hydroxydecalin, 1,2-, 1,3- und 1,4-Cyclohexandiol, 2,4-Dihydroxy-1,1,3,3-tetramethylcyclobutan, 1,4-Bis-(hydroxymethyl)-cyclohexan, Bis-(4-hydroxycyclohexyl)-methan, 2,2-Bis-(4-hydroxycyclohexyl)-propan, 2,4-Bis-(4-hydroxycyclohexyl)-2-methylpentan, Furfuryl- und Tetrahydrofurfurylalkohol, Bis-(hydroxymethyl)-norbornan, Bis-(hydroxymethyl)-tricyclodecan,
3. Araliphatische 1-4-wertige Alkohole des Molekulargewichtsbereichs 108 - 300, wie z. B. Benzylalkohol, Phenylethylalkohol, 3-Phenylpropanol oder 4,4'-Bis(2-hydroxyethyl)-diphenylmethan oder
4. 1 - 4 Hydroxylgruppen aufweisende Polythioether, Polyacetale, Polycarbonate oder insbesondere Polyester bzw. Polyether der in der Polyurethanchemie an sich bekannten Art mit mittleren Molekulargewichten von 250 bis 5000, vorzugsweise 300 bis 2000.
   Zu den in Betracht kommenden Polyesterpolyolen gehören beispielsweise die an sich bekannten, den gemachten Angaben entsprechenden Umsetzungsprodukte von zweiwertigen und gegebenenfalls zusätzlich dreiwertigen Alkoholen wie beispielsweise solchen der oben bereits unter 1, beispielhaft genannten Art mit unterschüssigen Mengen an mehrwertigen, vorzugsweise zweiwertigen Carbonsäuren oder deren Anhydriden wie beispielsweise Adipinsäure, Phthalsäure, Isophthalsäure, Phthalsäureanhydrid, Tetrahydrophthalsäureanhydrid, Hexahydrophthalsäureanhydrid, Maleinsäureanhydrid, und/oder dimeren oder trimeren Ölsäuren. Auch Hydroxylgruppen aufweisende Polycaprolactone der an sich bekannten Art können als Polyesterpolyole eingesetzt werden.
   Bei den Polyetherpolyolen handelt es sich um die an sich bekannten Alkoxylierungprodukte von geeigneten Startermolekülen, beispielsweise den oben unter 1, genannten mehrwertigen Alkoholen bzw. Gemischen derartiger Alkohole. Bei der Alkoxylierung werden vorzugsweise Ethylenoxid und/oder Propylenoxid allein, im Gemisch und/oder blockweise eingesetzt.

Die unter 1, genannten einfachen aliphatischen Alkohole, sowie die unter 4, genannten Polyester- bzw. Polyetherpolyole werden beim erfindungsgemäßen Verfahren bevorzugt eingesetzt.

Selbstverständlich können auch Mischungen der vorstehend genannten Hydroxylverbindungen eingesetzt werden. Dies ist sogar eine bevorzugte Ausführungsform des erfindungsgemäßen Verfahrens, da durch den Einsatz eines Gemisches von Hydroxylverbindungen unterschiedlicher Funktionalität, die Funktionalität des Allophanatgruppen enthaltenden Polyisocyanats in gewünschter Weise variiert werden kann.

Zur Herstellung der als Ausgangsmaterialien des erfindungsgemäßen Verfahrens dienenden Urethangruppen aufweisenden Verbindungen, sowie als Reaktionspartner für diese Urethangruppen aufweisenden Verbindungen kommen erfindungsgemäß vorzugsweise solche Diisocyanate der Formel

R₁(NCO)₂

zum Einsatz, für welche
- R₁: für einen aliphatischen Kohlenwasserstoffrest mit 2 bis 20, vorzugsweise 6 bis 10, Kohlenstoffatomen, einen cycloaliphatischen Kohlenwasserstoffrest mit 4 bis 20, vorzugsweise 6 bis 15 Kohlenstoffatomen oder einen Xylylenrest steht.

Beispiele derartiger Isocyanate sind 1,2-Diisocyanatoethan, 1,4-Diisocyanatobutan, 1,6-Diisocyanatohexan, 1,11-Diisocyanatoundecan, 2,2,4- und 2,4,4-Trimethyl-1,6-diisocyanatohexan, 1-Isocyanato-3-isocyanatomethyl-3,5,5-trimethylcyclohexan (Isophorondiisocyanat), 1,3-Diisocyanato-cyclobutan, 1,3- und 1,4-Diisocyanatocyclohexan, 4,4'-Bis-(isocyanatocyclohexyl)-methan, 1,2-Bis-(isocyanatomethyl)-cyclobutan, 1,3- und 1,4-Bis-(isocyanatomethyl)-cyclohexan, Hexahydro-2,4- und/oder 2,6-diisocyanatotoluol, Bis-isocyanatomethylnorbornan (Isomerengemisch), 1-Isocyanato-4(3)-isocyanatomethyl-1-methylcyclohexan oder p-Xylylendiisocyanat. Sowohl bei der Herstellung der erfindungsgemäß einzusetzenden Urethangruppen aufweisenden Verbindungen als auch als deren Reaktionspartner werden derartige Diisocyanate eingesetzt. Besonders bevorzugt sind 1,6-Diisocyanatohexan und Isophorondiisocyanat.

Bei der Herstellung der als Ausgangsmaterialien dienenden Urethangruppen aufweisenden Verbindungen, nicht jedoch als deren Reaktionspartner beim erfindungsgemäßen Verfahren können auch Monoisocyanate wie z. B. n-Hexylisocyanat oder Cyclohexylisocyanat verwendet bzw. mitverwendet werden, obwohl dies weniger bevorzugt ist.

Sowohl bei der Herstellung der als Ausgangsmaterialien dienenden Urethangruppen aufweisenden Verbindungen als auch als deren Reaktionspartner können auch höher als difunktionelle aliphatische bzw. cycloaliphatische Polyisocyanate verwendet bzw. mitverwendet werden. Beispiele derartiger Polyisocyanate sind die Isocyanuratgruppen aufweisenden Trimerisierungsprodukte von 1,6-Diisocyanatohexan oder von Isophorondiisocyanat.

Sowohl bei der Herstellung der Urethangruppen aufweisenden Ausgangsmaterialien als auch als deren Reaktionspartner können beliebige Gemische der genannten Isocyanate eingesetzt werden, mit der Einschränkung, daß man als Reaktionspartner für die Urethangruppen aufweisenden Verbindungen zweckmäßigerweise auf die Mitverwendung von Monoisocyanaten verzichten wird, da bei deren Mitverwendung die NCO-Funktionalität der erfindungsgemäßen Verfahrensprodukte herabgesetzt würde. Durch Wahl bestimmter Mischungsverhältnisse der Isocyanatkomponenten kann ebenso wie durch Wahl des Mischungsverhältnisses verschiedener Hydroxylverbindungen die Funktionalität der erfindungsgemäßen Verfahrensprodukte variiert werden.

Erfindungswesentlich ist die Mitverwendung von Zinnverbindungen bei der Umsetzung der Urethangruppen aufweisenden Verbindungen mit der Isocyanat-Komponente zu den entsprechenden Isocyanatgruppen aufweisenden Allophanaten.

Bei diesen Zinnverbindungen handelt es sich um Zinnsalze und Organozinnverbindungen. Bevorzugt sind dabei im Reaktionsgemisch lösliche Zinnverbindungen mit einem Zinngehalt von 10 bis 65 Gew.-% wie Zinn(II)- und Organozinnsalze organischer Säuren sowie Zinn(II)-halogenide. Beispiele der bevorzugten Zinnverbindungen sind Zinn(II)-chlorid, -bromid, -iodid, Zinn(II)-octanoat, Zinn-(II)-2-ethylhexanoat. Besonders bevorzugt sind dabei Zinn(II)-salze organischer Säuren wie beispielsweise Zinn(II)-n-octanoat bzw. Zinn(II)-2-ethylhexanoat.

Die Zinnverbindungen werden beim erfindungsgemäßen Verfahren in Mengen von 0,001 - 5,0, vorzugsweise 0,01 - 1,0 Gew.-% bezogen auf das Gesamtgewicht der Reaktionspartner eingesetzt.

Die Zinnverbindungen können dem Reaktionsgemisch nach beliebigen Methoden einverleibt werden. So ist es beispielsweise möglich, die Zinnverbindung bereits der Hydroxylgruppen aufweisenden Verbindung vor der Herstellung der Urethangruppen aufweisenden Verbindung zuzumischen. Ebenso ist es möglich, die Zinnverbindung, falls im Zweistufenverfahren gearbeitet wird, dem Reaktionsgemisch erst vor der Herstellung der Allophanatgruppen aufweisenden Verbindungen zuzumischen.

Zur Durchführung des erfindungsgemäßen Verfahrens werden im allgemeinen die Reaktionspartner in solchen Mengen eingesetzt, daß auf jede Urethangruppe der Urethangruppen aufweisenden Verbindung 2 bis 50, vorzugsweise 3 bis 12 Isocyanatgruppen der Polyisocyanat-Komponente, entfallen. Im Falle der Herstellung der Urethangruppen aufweisenden Verbindung in situ wird dementsprechend ein entsprechender Überschuß der Isocyanat-Komponente eingesetzt.

Die erfindungsgemäße Umsetzung erfolgt im allgemeinen im Temperaturbereich zwischen 50 und 140 °C. Der Verlauf der erfindungsgemäßen Umsetzung kann durch Bestimmung des NCO-Gehalts des Reaktionsgemisches verfolgt werden. Die Reaktion kann zu jedem beliebigen Zeitpunkt beispielsweise durch Abkühlen auf Raumtemperatur abgebrochen werden.

Bei der bevorzugten Variante des erfindungsgemäßen Verfahrens unter Herstellung der Urethangruppen aufweisenden Ausgangsverbindung in situ wird im allgemeinen wie folgt verfahren:

Das vorzugsweise als Isocyanat-Komponente eingesetzte Diisocyanat wird bei 50 - 80°C vorgelegt und die Hydroxyl-Komponente in flüssiger Form unter gutem Rühren eingetropft. Soll für die Urethanbildung und die Allophanatbildung das gleiche Isocyanat bzw. Isocyanatgemisch verwendet werden, so setzt man es am einfachsten von Anfang an in einem solchen Überschuß ein, daß das NCO/OH-Äquivalentverhältnis bei 3:1 und 12:1 liegt.

Nach erfolgter Urethanreaktion - kontrolliert durch Bestimmung des NCO-Gehaltes - wird die Temperatur auf 80 - 140 °C gesteigert und der Katalysator (die Zinnverbindung) zugegeben. Es wird so lange gerührt, bis der NCO-Gehalt auf den für vollständige Allophanatisierung errechneten Wert abgesunken ist.

Der Katalysator (die Zinnverbindung) kann jedoch auch zusammen mit dem Isocyanat vorgelegt oder gemeinsam mit der Hydroxylverbindung eindosiert werden.

Falls das Allophanatgruppen enthaltende Polyisocyanat von überschüssigem Diisocyanat befreit werden soll, geschieht dies entweder durch Dünnschichtdestillation oder durch fraktionierte Extraktion, beispielsweise unter Verwendung von n-Hexan oder Cyclohexan als Extraktionsmittel.

Bei der Durchführung des erfindungsgemäßen Verfahrens werden im allgemeinen Art und Mengenverhältnisse der Ausgangsmaterialien so gewählt, daß mindestens zwei Isocyanatgruppen aufweisende Allophanate, d.h. Allophanatpolyisocyan.ate, als Verfahrensprodukte entstehen. Derartige erfindungsgemäße Verfahrensprodukte zeichnen sich durch eine hervorragende Stabilität während der Dünnschichtbehandlung auch bei Temperaturen von 180 °C und mehr aus. Es treten nicht die bei Polyisocyanaten mit Uretdion- oder Biuretstruktur beobachteten Neben- und Äquilibrierungsreaktionen ein, die zu lästigen Anbackungen und zur Erhöhung der Viskosität führen.

Das erfindungsgemäße Verfahren eignet sich für eine kontinuierliche Durchführung. Hierbei werden zweckmäßigerweise mehrere Reaktoren in Form einer Kaskade hintereinandergeschaltet. In den ersten Reaktor werden kontinuierlich Diisocyanat, Hydroxylverbindung und Katalysator eindosiert. Durch Einstellung von Temperatur und Durchsatz wird erreicht, daß die Reaktion bei Verlassen des letzten Reaktors vollständig ist. Das Rohprodukt durchläuft anschließend einen Dünnschichtverdampfer, wo es von überschüssigem Diisocyanat befreit wird. Dieses wird in den ersten Reaktor zurückgeführt.

Die erfindungsgemäßen Verfahrensprodukte zeichnen sich durch eine niedrige Farbzahl, hohe Lichtechtheit und Farbstabilität sowie eine vergleichsweise niedrige Viskosität aus.

Insbesondere ist auch die ausgezeichnete Lagerstabilität der erfindungsgemäßen, von überschüssigem Ausgangsisocyanat befreiten Allophanatpolyisocyanate hervorzuheben. Die erfindungsgemäßen Verfahrensprodukte zeigen keinerlei Tendenz zur Abspaltung von monomerem Ausgangsisocyanat und unterscheiden sich insbesondere in diesem Punkt vorteilhaft von bekannten Uretdion- oder Biuretgruppen aufweisenden Polyisocyanaten.

Die erfindungsgemäßen Verfahrensprodukte stellen wertvolle Ausgangsmaterialien zur Herstellung von Polyurethankunststoffen nach dem Isocyanat-Polyadditionsverfahren, insbesondere zur Herstellung von Ein- oder Zweikomponenten-Polyurethanlacken dar. Bei Verwendung der erfindungsgemäßen Verfahrensprodukte in mit bekannten Blockierungsmitteln für Isocyanatgruppen blockierter Form eignen sich die erfindungsgemäßen Verfahrensprodukte insbesondere auch zur Herstellung von Polyurethan-Einbrennlacken.

Bevorzugte Reaktionspartner für die erfindungsgemäßen, gegebenenfalls in blockierter Form vorliegenden Verfahrensprodukte bei der Herstellung von Polyurethanlacken sind die in der Polyurethanlacktechnik an sich bekannten Polyhydroxypolyester, Polyhydroxypolyacrylate und gegebenenfalls niedermolekularen, mehrwertigen Alkohole. Geeignete derartige Reaktionspartner sind beispielsweise in DE-AS 2 304 893 beschrieben.

Die Mengenverhältnisse, in denen die erfindungsgemäßen, gegebenenfalls blockierten Polyisocyanate und die genannten Reaktionspartner bei der Herstellung von Polyurethanlacken umgesetzt werden, werden im allgemeinen so gewählt, daß auf eine (gegebenenfalls blockierte) Isocyanatgruppe 0,8 - 3, vorzugsweise 0,9 - 1,1 Hydroxyl-, Amino-, Mercapto- und/oder Carboxylgruppen entfallen.

Zur Beschleunigung der Aushärtung können in bekannter Weise die in der Isocyanatchemie üblichen Katalysatoren verwendet werden, wie z. B. tert. Amine wie Triethylamin, Pyridin, Methylpyridin, Benzyldimethylamin, N,N-Dimethylcyclohexylamin, N-Methylpiperidin, Pentamethyldiethylentriamin, N,N'-Endoethylenpiperazin, N,N'-Dimethylpiperazin usw., Metallsalze wie Eisen(III)-chlorid, Zinkchlorid, Zink-2-ethylhexanoat, Zinn(II)-2-ethylhexanoat, Dibutylzinndilaurat, Molybdänglykolat usw..

Die Allophanat-Polyisocyanate können auch in Einkomponentenlacken Verwendung finden. Durch Luftfeuchtigkeitshärtung werden glänzende hochwertige Beschichtungen erhalten. Gegebenenfalls können unterschüssige Mengen an OH-Komponenten, vor allem die bereits genannten OH-funktionellen Reaktionspartner, mitverwendet werden. Sie werden in einer Menge eingesetzt, daß auf eine OH-Gruppe mindestens 1,25, vorzugsweise 1,5 bis 10, NCO-Gruppen entfallen. Die genannten Katalysatoren können auch bei Einkomponenten-Lacken eingesetzt werden.

Bei Verwendung der Allophanat-Polyisocyanate in Einbrennlacken werden die NCO-Gruppen ganz oder teilweise in bekannter Weise blockiert. Das Polyisocyanat wird mit einem geeigneten Blockierungsmittel, vorzugsweise bei erhöhter Temperatur (z. B. 40 bis 140 °C) gegebenenfalls in Gegenwart eines geeigneten Katalysators, wie z. B. tert. Amine, Metallsalze wie Zink-2-ethylhexanoat, Zinn(II)-2-ethylhexanoat, Dibutylzinndilaurat oder Alkaliphenolat umgesetzt.

Geeignete Blockierungsmittel sind beispielsweise:

Monophenole wie Phenol, die Kresole, die Trimethylphenole, die tert. Butylphenole; tertiäre Alkohole wie tert.-Butanol, tert.-Amylalkohol, Dimethylphenylcarbinol; leicht Enole bildende Verbindungen wie Acetessigester, Acetylaceton, Malonsäurederivate wie Malonsäurediester mit 1 bis 8 C-Atomen in den Alkoholresten; sekundäre aromatische Amine wie N-Methylanilin, die N-Methyltoluidine, N-Phenyltoluidin, N-phenylxylidin; Imide wie Succinimid; Lactame wie ε-Caprolactam, δ-Valerolactam; Oxime wie Butanonoxim, Cyclohexanonoxim; Mercaptane wie Methylmercaptan, Ethylmercaptan, Butylmercaptan, 2-Mercaptobenzthiazol, α-Naphthylmercaptan, Dodecylmercaptan.

Zur Herstellung der Lackbindemittel werden gegebenenfalls blockiertes Polyisocyanat, polyfunktioneller Reaktionspartner, Katalysator und gegebenenfalls die üblichen Zusätze, wie z. B. Pigmente, Farbstoffe, Füllstoffe und Verlaufmittel miteinander mit Hilfe eines üblichen Mischaggregats, z. B. Dissolver, entweder mit oder ohne Lösungs- und Verdünnungsmittel gut vermischt und homogenisiert.

Die Anstrich- und Überzugsmittel können in lösungsmittelfreier flüssiger Form oder in Lösung oder aus der Schmelze, oder in fester Form nach den üblichen Methoden wie z. B. Streichen, Rollen, Gießen, Spritzen, dem Wirbelsinterverfahren oder dem elektrostatischen Pulversprühverfahren auf den zu beschichtenden Gegenstand aufgebracht werden.

Die die erfindungsgemäßen Polyisocyanate enthaltenden Lacke ergeben Filme, die überraschend gut auf metallischem Untergrund haften, besonders lichtecht, wärmefarbstabil und sehr abriebfest sind und, sofern sie in lufttrocknenden Lacken verwendet werden, besonders rasch, selbst bei Temperaturen um 0 °C antrocknen. Darüber hinaus zeichnen sie sich durch große Härte, Elastizität, sehr gute Chemikalienbeständigkeit, hohen Glanz, ausgezeichnete Wetterbeständigkeit und gute Pigmentierbarkeit aus.

Die folgenden Beispiele erläutern die Erfindung. Alle Prozentangaben betreffen Gewichtsprozente.

### Beispiele

### Beispiel 1:

Zu 336 g (2 mol) 1,6-Diisocyanatohexan (HDI) wurden 74 g (1 mol) n-Butanol bei 70°C zugetropft und eine Stunde gerührt, wobei ein NCO-Gehalt der Reaktionsmischung von 30,7 %, der der vollständigen Urethanisierung entspricht, erreicht wurde. Nach Erhöhen der Temperatur auf 100 °C wurden 0,1 g (0,02 % auf Reaktionsansatz) Zinn(II)-2-ethylhexanoat hinzugegeben. Man rührte 2 h bei 100 °C bis ein NCO-Gehalt von 20,5 % erreicht war, der vollständiger Allophanatisierung entspricht. Mit Hilfe der ¹³C-NMR-Spektroskopie wurden die Isocyanatfolgeprodukte in der Reaktionsmischung quantitativ erfaßt (s. Tabelle 1). Anschließend arbeitete man durch Dünnschichtdestillation auf, wobei 334 g (81,4 %) eines Produktes mit einem NCO-Gehalt von 12,9 %, einer Viskosität von 890 mPas bei 23 °C und einer Hazen-Farbzahl von 20 erhalten wurden.

### Vergleichsbeispiel 1 (katalysatorfrei, gemäß GB-PS 994 890)

336 g (2 mol) HDI und 74 g (1 mol) n-Butanol wurden bei 70 °C gerührt, bis ein NCO-Gehalt der Reaktionsmischung von 30,7 % (vollständige Urethanisierung) erreicht war. Dann erhöhte man die Temperatur auf 150 °C und rührte 24 h bei dieser Temperatur, wodurch ein NCO-Gehalt der Reaktionsmischung von 22,3 % erreicht wurde, der weitgehender Allophanatisierung entspricht. Mit Hilfe der ¹³C-NMR-Spektroskopie wurden die Isocyanatfolgeprodukte quantitativ erfaßt (s. Tabelle 1). Da die Reaktionsmischung stark gelb verfärbt war, wurde auf eine destillative Aufarbeitung verzichtet.

### Vergleichsbeispiel 2 (gemäß EP-B 0 000 194, Chlorwasserstoff als Katalysator):

336 g (2 mol) HDI und 74 g (1 mol) n-Butanol wurden bei 70 °C bis zum Urethan umgesetzt (NCO-Gehalt 30,7 %). Nach Erhöhen der Temperatur auf 100 °C wurden 4,1 g (1 %) Chlorwasserstoff (in Form einer 8 %igen Lösung von Chlorwasserstoff in HDI - Bildung des Carbamoylchlorids) hinzugegeben. Man rührte 5 h bei 100 °C, wobei ein NCO-Gehalt von 20,5 % erreicht wurde, der vollständiger Allophanatisierung entspricht. Die Reaktionsmischung wurde mit Hilfe der ¹³C-NMR-analysiert (Tabelle 1) und durch Dünnschichtdestillation aufgearbeitet. Dabei wurden 332 g (81 %) eines blaßgelben Produktes mit einem NCO-Gehalt von 12,9 %, einer Viskosität von 930 mPas bei 23 °C und einer Hazen-Farbzahl von 50 erhalten. Nach 2 Wochen Lagerung betrug die Hazen-Farbzahl 150.

**Tabelle 1**

| ¹³C-NMR-spektroskopische Analyse | | | |
|---|---|---|---|
| Struktur [Gew.-%] | Beispiel 1 | Vergleichsbeispiel | |
| | | 1 | 2 |
| Urethan | - | 19,2 | 10,5 |
| Allophanat | 97,8 | 65,0 | 82,4 |
| Uretdion | - | 3,3 | 1,6 |
| Isocyanurat | 2,2 | - | 1,6 |
| Harnstoff | - | 6,2 | 1,0 |
| Biuret | - | 6,3 | 3,0 |

¹³C-NMR-spektroskopische Analysen wurden mit Hilfe eines Bruker AMX-500-Spektrometers bei 125,76 MHz unter Proton Noise Decoupling (PND) durchgeführt. Als Lösungsmittel und ²H-"Lock" diente Dimethylsulfoxid-d₆, Tetramethylsilan (TMS) war interner Standard. Die Zuordnung der Strukturen erfolgte anhand der Carbonyl-C-Atom-Signale bei 148 - 160 ppm (gegenüber TMS). Die daraus erhaltenen molaren Anteile wurden auf Gew.-% umgerechnet.

### Beispiel 2:

In einem 2 l-Dreihalskolben wurden zu 1008 g (6 mol) 1,6-Diisocyanatohexan (HDI) bei 70 °C innerhalb von 30 min 75 g (0,5 mol) Triethylenglykol zugetropft. Nach weiteren 30 min bei 70 °C betrug der NCO-Gehalt der Reaktionsmischung 42,7 %, was vollständiger Umsetzung der OH-Gruppen zu Urethangruppen entspricht. Nach Erhöhen der Temperatur auf 100 °C wurden 0,3 g (0,03 %) Zinn(II)-2-ethylhexanoat hinzugegeben. Nach 3 Stunden bei 100 °C entsprach der NCO-Gehalt der Reaktionsmischung mit 38,8 % einer vollständigen Umsetzung der Urethangruppen zu Allophanatgruppen. Das Rohprodukt wurde einer Dünnschichtdestillation unterworfen. Es wurden 422 g eines nahezu farblosen Produktes (Hazen-Farbzahl 25) mit einer Viskosität von 1300 mPas bei 23 °C und einem NCO-Gehalt von 18,8 % erhalten. Das Produkt wurde gelchromatographisch auf seine Zusammensetzung hin untersucht (Tabelle 2).

### Vergleichsbeispiel 3 (gemäß GB-PS 994 890, Zink-Naphthenat als Katalysator wie in Beispiel 5 der GB-PS):

1008 g (6 mol) 1,6-Diisocyanatohexan (HDI) und 75 g (0,5 mol) Triethylenglykol wurden wie in Beispiel 2 bis zur vollständigen Urethanisierung umgesetzt (NCO-Gehalt 42,7 %). Anschließend versetzte man mit 1,15 g Zinknaphthenat. Innerhalb von 8 Stunden bei 50 °C sank der NCO-Gehalt auf 38,8 %. Nach Aufarbeitung durch Dünnschichtdestillation erhielt man ein braungelbes Produkt mit einem NCO-Gehalt von 20,4 % und einer Viskosität bei 25 °C von 1350 mPas.
GPC-Analyse siehe Tabelle 2.

### Vergleichsbeispiel 4 (gemäß GB-PS 994 890, Tertiäres Amin als Katalysator wie in Beispiel 4 der GB-PS):

Wie in vorstehendem Vergleichsbeispiel 3 wurde die Urethanlösung bereitet und dann mit 1,15 g Diazabicyclooctan versetzt und 24 h bei 70 °C gerührt. Der theoretische NCO-Abfall wurde aber erst nach weiteren 16 h bei 120 °C erreicht. Nach Aufarbeitung wurde ein tiefgelbes Öl der Viskosität von 1050 mPas bei 25 °C mit einem NCO-Gehalt von 20,2 % erhalten.
GPC-Analyse siehe Tabelle 2.

**Tabelle 2**

| Gelchromatographische Analyse | | | |
|---|---|---|---|
| Komponente [Flächen-%] | Beispiel 2 | Vergleichsbeispiel | |
| | | 3 | 4 |
| HDI | 0,2 | 0,7 | 0,5 |
| Dimeres Diisocyanat | 0,5 | 1,5 | 6,3 |
| Trimeres Diisocyanat | 2,9 | 10,6 | 11,3 |
| Bisurethan aus 1 mol Triethylenglykol und 2 mol Diisocyanat | 1,0 | 10,3 | 10,3 |
| Monourethan-Monoallophanat aus 1 mol Triethylenglykol und 3 mol Diisocyanat | 1,6 | 9,8 | 15,6 |
| Bisallophanat aus 1 mol Triethylenglykol und 4 mol Diisocyanat | 31,9 | 20,8 | 12,2 |
| Summe aller Polymer-homologer Verbindungen | 61,9 | 46,3 | 43,8 |

### Beispiele 3 - 13: siehe Tabelle 3 und 4

**Tabelle 3:**

| Beispiele 3 - 8 (analog Beispiel 1): | | | | | | |
|---|---|---|---|---|---|---|
| Beispiel | 3 | 4 | 5 | 6 | 7 | 8 |
| Isocyanat | HDI | HDI | HDI | HDI | HDI | HDI |
| Alkohol | n-BuOH | n-BuOH | n-BuOH | n-BuOH | n-BuOH | n-BuOH |
| NCO/OH | 4,0 | 6,0 | 8,0 | 10,0 | 4,0 | 10,0 |
| Katalysator | A | A | A | A | B | B |
| [%] | 0,04 | 0,02 | 0,02 | 0,02 | 0,05 | 0,05 |
| Reaktionstemperatur [°C] | 110 | 110 | 110 | 110 | 110 | 110 |
| Reaktionszeit [h] | 2 | 3 | 2 | 1,5 | 4 | 1,5 |
| NCO_{End} [%] | 20,3 | 29,1 | 33,8 | 36,7 | 20,8 | 36,6 |
| Ausbeute_{Harz} [%] | 80,7 | 58,9 | 46,0 | 38,9 | 76,9 | 39,2 |
| NCO_{Harz} [%] | 12,7 | 15,3 | 17,4 | 18,6 | 12,5 | 17,8 |
| Viskosität [mPas] | 870 | 200 | 160 | 140 | 1050 | 240 |
| Hazen-Farbzahl | 25 | 25 | 30 | 25 | 40 | 40 |
| Katalysator: A = Zinn(II)-2-ethylhexanoat B = Zinn(II)chlorid | | | | | | |

**Tabelle 4:**

| Beispiele 9 - 13 (analog Beispiel 1): | | | | | |
|---|---|---|---|---|---|
| Beispiel | 9 | 10 | 11 | 12 | 13 |
| Isocyanat | HDI | HDI | IPDI | IPDI | IPDI |
| Alkohol | MeOH | EtOH | n-BuOH | n-BuOH | n-BuOH |
| NCO/OH | 8,0 | 8,0 | 6,0 | 8,0 | 10,0 |
| Katalysator | A | A | A | A | A |
| [%] | 0,05 | 0,05 | 0,1 | 0,1 | 0,1 |
| Reaktionstemperatur [°C] | 110 | 110 | 110 | 110 | 110 |
| Reaktionszeit [h] | 2 | 2,5 | 6 | 3,5 | 3 |
| NCO_{End} [%] | 34,9 | 35,0 | 23,1 | 26,2 | 28,0 |
| Ausbeute_{Harz} [%] | 48,1 | 47,2 | 56,8 | 41,9 | 38,0 |
| NCO_{Harz} [%] | 18,6 | 18,4 | 13,4 | 13,8 | 14,2 |
| Viskosität [mPas] | 540 | 270 | 230* | 200* | 180* |
| Hazen-Farbzahl | 30 | 30 | 50 | 40 | 50 |

| | | | | | |
|---|---|---|---|---|---|
| * = 70%ige Lösung in Methoxypropylacetat Katalysator: A = Zinn(II)-2-ethylhexanoat B = Zinn(II)-chlorid | | | | | |

## Patentansprüche

1. Verfahren zur Herstellung von aliphatisch und/oder cycloaliphatisch gebundene Isocyanatgruppen aufweisenden Allophanaten durch Umsetzung von Urethangruppen aufweisenden organischen Verbindungen mit organischen Polyisocyanaten mit aliphatisch und/oder cycloaliphatisch gebundenen Isocyanatgruppen, bei einer Temperatur von 80 - 140°C, wobei das Allophanatgruppen enthaltende hergestellte Polyisocyanat durch Dünnschichtdestillation oder fraktionierte Extraktion von überschüssigem Diisocyanat befreit wird, dadurch gekennzeichnet, daß diese in Gegenwart von Zinn(II)-salzen, Zinn(II)-halogeniden und Zinn(II)-salzen organischer Säuren hergestellt wurden

2. Verfahren gemäß Anspruch 1, dadurch gekennzeichnet, daß man als Zinn(II)-Verbindungen im Reaktionsgemisch lösliche Zinnverbindungen mit einem Zinngehalt von 10 bis 65 Gew.-% in einer Menge von 0,001 bis 5 Gew.-%, bezogen auf das Gesamtgewicht der Reaktionspartner, verwendet.

3. Verwendung der gemäß Anspruch 1 hergestellten Allophanatgruppen aufweisenden Polyisocyanate als Aufbaukomponente bei der Herstellung von Polyurethankunststoffen, insbesondere als, gegebenenfalls in blockierter Form einzusetzendes, Vernetzerharz für Zweikomponenten-Polyurethanlacke.

## Claims

1. Process for the production of allophanates containing aliphatically and/or cycloaliphatically bound isocyanate groups by reaction of organic compounds containing urethane groups with organic polyisocyanates having aliphatically and/or cycloaliphatically bound isocyanate groups at a temperature of 80-140°C, wherein excess diisocyanate is removed from the resultant polyisocyanate containing allophanate groups by film distillation or fractional extraction, characterised in that these allophanates were produced in the presence of tin(II) salts, tin(II) halides and tin(II) salts of organic acids.

2. Process according to claim 1, characterised in that tin compounds soluble in the reaction mixture having a tin content of 10 to 65 wt.% are used as the tin(II) compounds in a quantity of 0.001 to 5 wt.%, relative to the total weight of the reactants.

3. Use of the polyisocyanates containing allophanates produced according to claim 1 as a synthesis component in the production of polyurethane plastics, in particular as a crosslinking resin, optionally used in blocked form, for two-component polyurethane lacquers.

## Revendications

1. Procédé pour la préparation d'allophanates présentant des groupes isocyanate liés à des radicaux aliphatiques et/ou cycloaliphatiques, par mise en réaction de composés organiques présentant des groupes uréthane avec des polyisocyanates organiques contenant des groupes isocyanate liés à des radicaux aliphatiques et/ou cycloaliphatiques, à une température de 80 à 140°C, dans lequel on libère du diisocyanate en excès le polyisocyanate préparé contenant des groupes allophanate, par distillation en couche mince ou par extraction fractionnée, caractérisé en ce qu'on les prépare en présence de sels d'étain(II), d'halogénures d'étain(II) et de sels d'étain(II) d'acides organiques.

2. Procédé selon la revendication 1, caractérisé en ce qu'on utilise, à titre de composés d'étain(II), des composés d'étain solubles dans le mélange réactionnel possédant une teneur en étain de 10 à 65% en poids, en une quantité de 0,001 à 5% en poids rapportés au poids total des partenaires réactionnels.

3. Utilisation des polyisocyanates présentant des groupes allophanate préparés conformément à la revendication 1, à titre de constituant lors de la préparation de matières synthétiques de polyuréthanne, en particulier à titre de résine de réticulation, le cas échéant à mettre en oeuvre sous forme bloquée, pour des laques, des vernis ou des peintures de polyuréthanne à deux composants.
